# EUROPEAN PATENT APPLICATION

(11) **EP 3 906 908 A1**
(43) Date of publication of application: **10.11.2021**
(21) Application number: 20173436.5
(22) Date of filing: 07.05.2020
(51) Int. Cl.: A61F 13/47, A61F 13/49, A61F 13/505

(54) **ABSORBENT ARTICLES HAVING INTEGRATED STRETCH MONITORING**

(71) Applicant: Ontex BV, 9255 Buggenhout (BE); Ontex Group NV, 9320 Erembodegem (BE)
(72) Inventor: Weber, Ainas, 53474 Bad Neuenahr-Ahrweiler (DE); Hellmold, Jens, 59269 Beckum (DE); Idelson, Alissa, 53359 Rheinbach (DE); Heirman, Lisa, 9255 Buggenhout (BE)
(74) Representative: Macchetta, Andrea

(57) **Abstract**

An absorbent article for personal hygiene such as a diaper, or training pant, or incontinence insert, the absorbent article comprising: a liquid permeable topsheet; a liquid impermeable backsheet; an absorbent core interposed between said topsheet and said backsheet, together forming an absorbent article chassis having a front edge, a back edge and oppositely disposed side edges connecting said front and back edges and running along a longitudinal axis y; an elastic-material-comprising component selected from the group consisting of: one or more cuffs extending substantially parallel to said side edges comprising one or more elastic elements; one or more transversal barriers extending substantially perpendicular to the longitudinal axis y and along said front and/or back edges, said transversal barriers comprising one or more elastic elements; one or more, preferably at least two, side panels comprising one or more elastic elements, said side panels being joined to said chassis about a proximal end thereof and having a distal end extending away from said chassis along a transverse axis x being substantially perpendicular to said longitudinal axis y; one or more elastic waistbands positioned adjacent said front and/or back edges comprising one or more elastic elements; and combinations thereof; and a detection device that can be removably attached to the absorbent article, wherein the elastic elements comprise a conductive material and/or coating and are in electrical communication with said detection device arranged to provide a signal upon a change in resistance or capacitance in response to a change in extension of said elastic element(s) (107).

## Description

### TECHNICAL FIELD

The disclosure relates to absorbent articles such as baby or adult diapers, pants, or incontinence briefs for adults and/or children. Typically said absorbent articles comprising added electronics adapted to monitor appropriate fit of said absorbent articles.

### BACKGROUND

Absorbent articles for personal hygiene are designed to absorb and contain bodily exudates, such as a large quantity of urine. Non-limiting examples of disposable absorbent articles include diapers, pants, training pants, pADL, adult incontinence products, and feminine hygiene products (including, for example, sanitary napkins and tampons). Other examples of disposable absorbent articles include bandages and wound dressings. In some embodiments, for example, an absorbent article comprises several layers providing different functions, for example a topsheet, a backsheet and in-between an absorbent core, among other layers.

The function of the absorbent core is to absorb and retain the exudates for a prolonged amount of time, for example overnight for a diaper, minimize re-wet to keep the wearer dry and avoid soiling of clothes or bed sheets. The majority of currently marketed absorbent articles comprise as absorbent material a blend of comminuted wood pulp with superabsorbent polymers (SAP) in particulate form, also called absorbent gelling materials (AGM), see for example U.S. Pat. No. 5,151,092 (Buell). Absorbent articles having a core consisting essentially of SAP as absorbent material (so called "airfelt-free" cores) have also been proposed but are less common than traditional mixed cores (see e.g. WO2008/155699 and WO2012/052172).

A number of disclosures exist related to the use of indicia indicating whether certain components, such as elastic laminates like elastic ears or panels, are correctly tensioned and applied, such as for example as described in EP2346461A1, EP1959899A2, and EP2854721A1. All such systems however rely on visual indicators or indicia that helps the user or caregiver understand whether the absorbent article such as a diaper is correctly fitted and elastic components sufficiently tensioned. Drawbacks associated to these principles include lesser accuracy in tension control and monitoring.

A need exists for an inexpensive and yet effective means to automatically determine stretch and tension of elastic components in order to inform the user and/or caregiver about correct fitting of the absorbent article onto the wearer.

A need further exists for such means to also provide other functional automated monitoring such as exudate detection monitoring and/or risk of leakage.

### SUMMARY

In a first aspect, the disclosure relates to an absorbent article for personal hygiene such as a diaper, or training pant, or incontinence insert, the absorbent article comprising: a liquid permeable topsheet; a liquid impermeable backsheet; an absorbent core interposed between said topsheet and said backsheet, together forming an absorbent article chassis having a front edge, a back edge and oppositely disposed side edges connecting said front and back edges and running along a longitudinal axis y; an elastic-material-comprising component selected from the group consisting of: one or more cuffs extending substantially parallel to said side edges comprising one or more elastic elements; one or more transversal barriers extending substantially perpendicular to the longitudinal axis y and along said front and/or back edges, said transversal barriers comprising one or more elastic elements; one or more, preferably at least two, side panels comprising one or more elastic elements, said side panels being joined to said chassis about a proximal end thereof and having a distal end extending away from said chassis along a transverse axis x being substantially perpendicular to said longitudinal axis y; one or more elastic waistbands positioned adjacent said front and/or back edges comprising one or more elastic elements; and combinations thereof; and a detection device that can be removably attached to the absorbent article, wherein the elastic elements comprise a conductive material and/or coating (and/or the elastic elements are arranged in conjuction with conductive elements that are substentially adjacent said elastic elements) and are in electrical communication with said detection device arranged to provide a signal upon a change in resistance or capacitance in response to a change in extension of said elastic element(s) (107).

In a second aspect, the disclosure relates to a kit of parts comprises a washable and re-usable outer shell, a plurality of disposable inserts, and at least one detection device.

### BRIEF DESCRIPTION OF THE FIGURES

**Fig. 1A-C** illustrates a schematic of an article assembly according to an embodiment of the disclosure.
**Fig. 2** illustrates a schematic of an insert according to an embodiment of the disclosure.
**Fig. 3** illustrates a schematic of an insert according to an embodiment of the disclosure.
**Fig. 4** illustrates a schematic of an article shell according to an embodiment of the disclosure.
**Fig. 5** illustrates a schematic of an absorbent article according to an embodiment of the disclosure.
**Fig. 6** illustrates a schematic of an elastic element comprising an electrically conductive yarn and/or filamenet according to an embodiment of the disclosure.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed disclosure. However, it is to be understood that the value to which the modifier "about" refers is itself also specifically disclosed.
"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.
The expression "% by weight" (weight percent), here and throughout the description unless otherwise defined, refers to the relative weight of the respective component based on the overall weight of the formulation.
The recitation of numerical ranges by endpoints includes all numbers and fractions subsumed within that range, as well as the recited endpoints unless otherwise stated.
"Bonded" refers to the joining, adhering, connecting, attaching, or the like, of at least two elements. Two elements will be considered to be bonded together when they are bonded directly to one another or indirectly to one another, such as when each is directly bonded to intermediate elements.
The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.
The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).
"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.
As used herein, the "body-facing" or "bodyside" or "skin-facing" surface means that surface of the article or component which is intended to be disposed toward or placed adjacent to the body of the wearer during ordinary use, while the "outward", "outward-facing", "garment-facing" or "garment-side" surface is on the opposite side, and is intended to be disposed to face away from the wearer's body during ordinary use. Such outward surface may be arranged to face toward or placed adjacent to the wearer's undergarments when the absorbent article is worn.
As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.
The term "nonelastic" or "inelastic" refers to any material which does not fall within the definition of "elastic" above.

Embodiments of the articles and processes according to the disclosure will now be described. It is understood that technical features described in one or more embodiments maybe combined with one or more other embodiments without departing from the intention of the disclosure and without generalization therefrom.

### THE ARTICLE

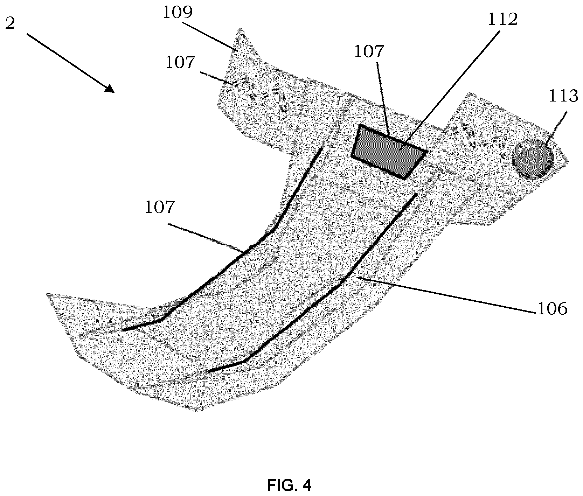

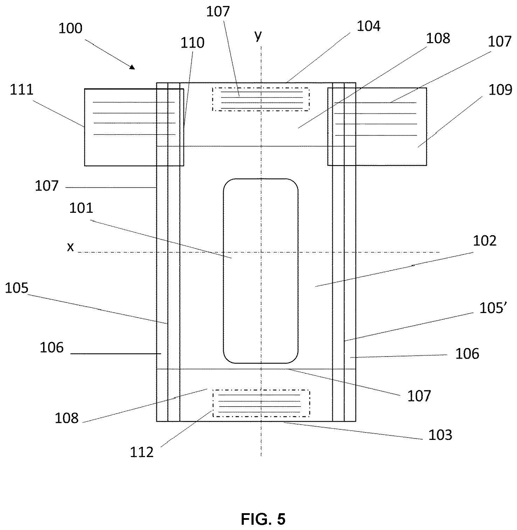

Absorbent articles herein, such as in Figs. 4-5, are for personal hygiene such as a diaper (100), or training pant (1), or incontinence insert (7), the absorbent article typically comprises: a liquid permeable topsheet (8); a liquid impermeable backsheet (9); an absorbent core (10, 101) interposed between said topsheet (8) and said backsheet (9), together forming an absorbent article chassis (102) having a front edge (103), a back edge (104) and oppositely disposed side edges (105, 105') connecting said front and back edges (103, 104) and running along a longitudinal axis (y); an elastic-material-comprising component selected from the group consisting of: one or more cuffs extending substantially parallel to said side edges (105, 105') comprising one or more elastic elements (107); one or more transversal barriers (108) extending substantially perpendicular to the longitudinal axis (y) and along said front and/or back edges (103, 104), said transversal barriers (108) comprising one or more elastic elements (107); one or more, preferably at least two, side panels (109) comprising one or more elastic elements (107), said side panels being joined to said chassis (102) about a proximal end (110) thereof and having a distal end (111) extending away from said chassis (102) along a transverse axis (x) being substantially perpendicular to said longitudinal axis (y); one or more elastic waistbands (112) positioned adjacent said front and/or back edges (103, 104) comprising one or more elastic elements (107); and combinations thereof; and a detection device (113) that can be removably attached to the absorbent article, wherein the elastic elements (107) comprise a conductive material and/or coating (and/or the elastic elements are arranged in conjuction with conductive elements that are substentially adjacent said elastic elements) and are in electrical communication with said detection device (113) arranged to provide a signal upon a change in resistance or capacitance in response to a change in at least one of extension, tension, and/or rupture, of said elastic element(s) (107). Advantageously, the extension of elastics may be accurately monitored by change in electrical properties such as resistance therealong. Indeed, without wishing to be bound by theory, as the elastics stretch the electrical resistance will increase and the detection device may detect these changes and provide a signal to for example a mobile device once the resistance reaches different predetermined thresholds to provide an accurate indication of stretch and thus fit of the absorbent article onto the wearer.

Another advantage, in the case where the elastic material reacts with oil or cream and becomes brittle, the tension (i.e. retractive force) is decreasing over time. The above arrangement allows to detect when the elastic is close to breakage or indeed when it does break.

In an embodiment, the stretch measurement can be beneficial for putting on the absorbent article using the right level of tesnsion e.g. on the elastic ears, i.e. avoid pulling too hard but also avoid too loose fit which may result in sagging.

In an embodiment, while the article is worn, which can be for several hours, continuous monitoring of the stretch level may indicate whether good fit of the article is maintained over time. For instance, it has been observed that under continued tension in combination with elevated temperature (body heat), some elastic ear materials suffer from a loss of retractive force which may result in sagging of the absorbent article.

In an embodiment, it has been observed that some oils, creams and lotions used for baby skincare can chemically react with certain elastic materials, by which the retraction force is negatively affected or even the material integrity is deteriorated to the extent that in extreme cases elastic ears could even tear off from the chassis. The latter is not only inconvenient in terms of product fit but also represents a safety risk, as e.g. a baby might grab and swallow a piece of material that has come off. Monitoring the strech level e.g. of the elastic ears could provide an early warning in such cases so that the artcile is changed before the fit deteriorates or even a component comes off.

In an embodiment, the change in the stretch level around the belly is used as an indication for the voiding event. This may result in an advice for checking/changing the diaper provided by the detection device.

In an embodiment, the detection device comprises a battery, a processor, a transmitter and optionally a memory, and typically wherein the detection device is adapted to compare a detected value (typically relating to one or more electrical properties such as the resistance within one or more elastics) and compare it to a plurality of pre-set thresholds and wherein a signal is triggered by the detection device each time the detected value is equal to said pre-set thresholds and wherein each of the plurality of pre-set thresholds correspond to different stretch conditions (for example different %extensions of the elastic component).

In an embodiment, the detection device is adapted to apply an alternating current through the conductive material and/or coating. Advantageously, this allows for detection of presence of exudates through impedance changes of the conductive material and/or coating.

The detection device may further comrise a plurality of conductive terminals that are arranged to come into electrical communication with the conductive material and/or coating either directly or indirectly (e.g. via further conductive ink tracks as will be further described in more detail hereinbelow). The detection device may be in data communication (such as via near-field-communication or Bluetooth) with a mobile device or other receiver. Alternatively or in addition, the detection device may comprise a display arranged to display information or indicia relating to a plurality of different status conditions such as battery status, stretch status of the elastic components, and/or warnings such as a warning related to leakage risk and/or battery close to depletion.

In an embodiment, the detection device is further arranged to provide a signal upon a change in resistance or capacitance in response to an exudate coming in contact with said elastic element(s) (107) typically following a voiding event. Advantageously, this arrangement allows for simple and accurate detection of risk of leakage by use of the same componenets whithout additional complex hardware needing to be integrated therein. Indeed, the risk of leakage is greatest once exudates make their way to extremities of absorbent articles such as the cuffs, the waistband, the elastic panels (also referred to as elastic back ears in the art), and/or transversal barriers, and adding elastics that provide in addition to their beneficial elastic properties, conductive properties allow to detect when exudates reach said extremities since this then generally triggers a change in conductive properties that may be detected by the detection device to provide a warning to the caregiver in response thereto.

In an embodiment the elastic elements herein are selected from the group consisting of one or more elastic strands (such as Lycra elastics), an elastic film, and combinations thereof.

In an embodiment, the elastic elements herein comprise more than one conductive component, preferably at least two, typically two, conductive components, wherein each component comprises a different material such that each said component(s) changes electrical properties differently from the other component(s) upon presence of exudates. Preferably wherein at least one conductive component changes electrical properties upon chemical contact thereto, and at least one other conductive component changes electrical properties upon elongation thereof by mechanical impact with the exudate. Typically wherein each components triggers a response signal at different points in time.

In an embodiment, the conductive material and/or coating comprises a material selected from the group consisting of graphene, graphite, poly(pyrrole)s (PPY), polyanilines (PANI), poly(thiophene)s (PT), poly(p-phenylene sulfide) (PPS), poly(p-phenylene) (PPP), Poly(acetylene)s (PAC), Poly(p-phenylene vinylene) (PPV), poly(3,4-ethylenedioxythiophene) (PEDOT), and mixtures thereof, more preferably poly(3,4-ethylenedioxythiophene) polystyrene sulfonate (PEDOT:PSS). Advantageously, these materials allow to avoid the inclusion of metals within the absorbent article, the latter being considered undesirable due to their environmental impact and difficulty to recycle or due to their limited biocompatibility if they need to come in contact with the skin.

Preferably, the material is comprised at a level of from 10 to 45%, preferably from 15 to 35%, by weight of the total composition of the conductive material and/or coating. It has been found that incorporating such materials at these levels allows for sufficient detectability in changes to electrical properties such as resistance and/or capacitance.

In an embodiment, a conductive ink pattern is applied, preferably printed, onto at least a portion of the chassis (102), preferably a skin-facing surface of the backsheet (9), and arranged such to form a conductive bridge between said elastic elements (107) and the detection device (113). This advantageously permits to place or position the detection device at a distance from the elastic materials (such as at a belly position at the front of the article) such to make it more convenient and less likely to be knocked out of place during movement of the wearer, whilst still ensuring detectability of the exudates.

Preferably, the conductive ink pattern comprises an adhesive or the adhesive as described herein may be applied instead of said conductive ink pattern, preferably a stretchable adhesive. Examples of suitable commercially available stretchable adhesives are manufactured and sold by H.B. Fuller, 1200 Willow Lake Boulevard, P.O. Box 64683, St. Paul, MN 55164-0683, under the brand Conforma® adhesives, such as Conforma 9800. Such stretchable adhesives are typically multilayered and have at least three, preferably at least five layers. Conductive materials listed above may be included in at least a portion of the adhesive (such as at least one of the layers) to provide conductive properties thereto. Advantageously, combining electric properties to such adhesives allow for accurate detection even upon repeated stretching whilst the articles are worn and the wearer moves in different positions.

Preferably, the conductive ink comprises a material selected from the group consisting of graphene, graphite, poly(pyrrole)s (PPY), polyanilines (PANI), poly(thiophene)s (PT), poly(p-phenylene sulfide) (PPS), poly(p-phenylene) (PPP), Poly(acetylene)s (PAC), Poly(p-phenylene vinylene) (PPV), poly(3,4-ethylenedioxythiophene) (PEDOT), and mixtures thereof, more preferably poly(3,4-ethylenedioxythiophene) polystyrene sulfonate (PEDOT:PSS).

In an embodiment, the one or more elastic elements (107) are strand coated with said conductive material and/or coating. Advantageously this allows to impart conductive properties whilst retaining softness to the touch by limiting the amount of surface area covered by such coatings/materials. By strand coated it is meant that each of the elastic elements are individually strand coated with a strand coating of adhesive. In one embodiment, a Nordson Surewrap™ adhesive technology is used to strand coat the elastic elements. Various coating methods and techniques, including strand coating methods and techniques, are shown for example in U.S. Patent Nos. 5,340,648, 5,501,756, 5,507,909, 6,077,375, 6,200,635, 6,235,137, 6,361,634, 6,561,430, 6,520,237, 6,582,518, 6,610,161, 6,613,146, 6,652,693, 6,719,846 and 6,737,102.

In an embodiment, the detection device (113) comprises an integrated memory to store at least a portion of the data collected by a processor contained within the detection device (113) during a monitoring period (i.e. the wearing of the article), and a transmitter to transmit said data at regular predetermined periods of time to a data receiver for further processing of said data.

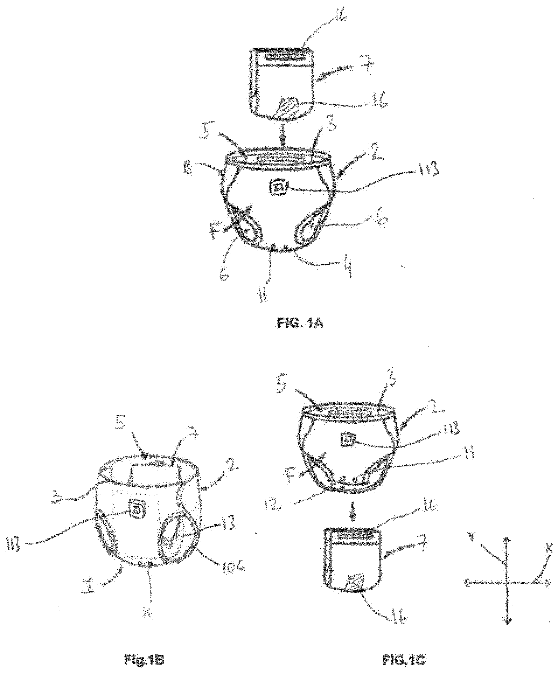

In an embodiment, as exemplified in Fig.1 and Fig.4, the absorbent article comprises a disposable insert (107) and a washable and re-usable outer shell (2), wherein the disposable insert (7) comprises the liquid permeable topsheet, the liquid impermeable backsheet and the absorbent core, and wherein the washable and re-usable outer shell (2) comprises a second liquid permeable woven or nonwoven backsheet (4) to which the elastic-material-comprising components are joined. Advantageously this allows for an environmentally and cost-friendly execution where the electronic components are re-used and only the absorbent insert free of electronics is disposed of.

Alternatively, the absorbent article comprises a disposable insert (107) and a washable and re-usable outer shell (2), wherein the disposable insert (7) comprises the liquid permeable topsheet, a liquid permeable backsheet and the absorbent core positioned between said permeable topsheet and permeable backsheet, and wherein the washable and re-usable outer shell (2) comprises a second backsheet being the liquid impermeable backsheet (4) to which the elastic-material-comprising components are joined. Advantageously, composting of the insert is made easier by the absence of a liquid impermeable layer (typically requiring the use of polymer based films) and the impermeable backsheet is rather integrated in the re-usable outer shell that may be washed after use.

Preferably, the disposable insert (107) further comprises a pair of longitudinally extending inner cuffs (13) and wherein the re-usable outer shell comprises a second pair of longitudinally extending outer cuffs (106) that are positioned outboard the inner cuffs (13) in assembled state, preferably wherein the outer cuffs (106) comprise the elastic elements (107) comprising a conductive material and/or coating, and preferably wherein the disposable insert is free of conductive material and/or coating.

Preferably the second liquid permeable backsheet (4), preferably a garment facing surface of said second liquid permeable backsheet, comprises the conductive ink pattern described hereinabove, preferably wherein said pattern extends from a front edge to a back edge of said backsheet.

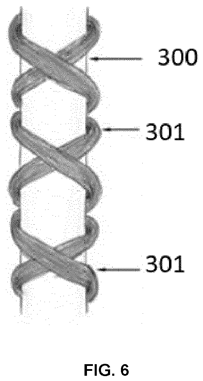

In an embodiment, the elastic element (107) comprises an electrically conductive yarn or fiber. As illustrated in Fig. 6, the elstic element (107) may be wrapped with one or more electrically conductive yarns or fibers (300) (i.e. a conductive wrap) typically such that said yarns or fibers form a spiral around the elastic element whenerein voids (301) are formed where no electrically conductive material is present. Advantegeeously this allows conductive bridges to be formed and a change in resistance to be detectable by the detection device when exudates fill the voide (301) and/or this allows the stretchable elastic element to stretch, whilst the non-stretchable conductive material follows along with the stretchable element when it deforms.

In an embodiment, the conductive yarns or fibers are substantially inelastic and the elastic element is arranged to stretch and elongate within the spiral(s) of yarns or fibers such that said spiral(s) are permanently deformed upon, preferably each, such elongation. Advantageously this arrangement allows for a non-repeatable signal to be generated in response to the incremental permanent deformation of the conductive wrap.

Electro-conductive (or electrically conductive) yarns can be produced in various ways and can be arranged to obtain very different properties in terms of conductivity, touch, as well as strength and elasticity. For example, elastic and electro-conductive yarns (el2-yarns) can be manufactured via hollow spindle spinning. For example, a rubber-based elastic core is formed around which electro-conductive winding yarns, based on silver, copper and stainless steel, are wound.

The yarn or fiber may comprise metal (the metal is preferably selected from silver, copper, stainless steel and combinations thereof), although generally less preferred unless they form part of a re-usable component as described herein. Preferably, such metal containing conductive wraps are used in combination with a re-usable shell of an absorbent article as described herein generally for improved environmental impact.

Alternatively, the yarns or fibers may be directly included within a core of the elastic element.

In an embodiment, the elastic element (107) comprises a conductive filler, preferably wherein the conductive filler has a melting temperature that is higher than the melting temperature of one or more polymers forming said elastic element.

Preferably, the conductive filler is selected from the group consisting of graphene, graphite, and mixtures thereof, preferably wherein said filler is comprised at a level of from 5% to 30%, preferably from 10% to 25%, by weight of the elastic element.

In an embodiment, the absorbent core comprises a core wrap enclosing absorbent material therein and wherein a top layer of said core wrap is joined to a bottom layer of said core wrap to form one or more channels and preferably wherein the conductive ink printed onto the second liquid permeable backsheet substantially follows a shape of said channels.

In an embodiment, a kit of parts is provided that comprises a washable and re-usable outer shell (2) as described herein, a plurality of disposable inserts (7) as described herein and at least one detection device (113).

In an embodiment, as exemplified in Figs.1A-C, the absorbent article (herein also referred to as the absorbent assembly (1)) may comprise: an elastic outer cover (2) comprising a front (F) and back (B) waist region (3) having uppermost and lowermost edges, and a crotch region (4) interposed between the front and back waist region, wherein the uppermost edges of the waist region (3) form a waist opening (5) and the lowermost edges of the waist region (3) together with lateral extremities of the crotch region (4) form two oppositely disposed leg openings (6); and a disposable absorbent insert (7) comprising a liquid permeable topsheet (8), a liquid impermeable backsheet (9), and an absorbent core (10) interposed between said topsheet (8) and said backsheet (9), wherein a skin-facing surface of said outer cover (2) comprises one or more fastening surfaces and wherein a garment-facing surface of the absorbent insert (7) comprises one or more fastening surfaces arranged to releasably engage with the one or more fastening surfaces of said outer cover (2), and wherein said crotch region (4) comprises one or more re-fastenable members (11) such that said crotch region (4) is unitary when said re-fastenable members (11) are fastened and that said crotch region (4) is separated into two crotch halfs when said re-fastenable members (11) are unfastened to form a crotch-release opening (12), and in that the disposable absorbent insert (7) may be single-handedly removed from said elastic outer cover (2) from said crotch-release opening (12). Advantageously, this allows a subject to remove the insert without soiling the elastic outer cover that may be re-used by inserting a new insert therein, even more advantageously this operation may be carried out with a single hand thus allowing the subject to handle the baby with the other hand and or doing other operations concurrently. It is understood that this embodiment may comprise elastic elements comprising conductive materials and/or coatings as described in embodiments hereinabove.

In a preferred embodiment, the detection device (113) described herein my be removably attached to a garment facing surface of the outer cover (2) herein. The detection device may comprise a mechanical fastener to couple to the outer cover (2), for example in the form of hooks arranged to hook onto loops formed on the outer cover, or one or more clips that mechanically retain the detection device within a respective pocket that provides access to the conductive ink pattern as described herein so as to form an electric connection thereto.

In an embodiment, the re-fastenable members (11) are mechanical fasteners, preferably in the form of more than one snap fasteners. Such allow for multiple secure opening/re-closing over time for effective re-usability of the outer cover.

The outer cover may be constructed with woven textile material comprising one or more elastics and/or comprising an elastic fabric that provides it with stretch properties similar to underwear for added comfort. The outer cover may further be washable.

In an embodiment, the elastic outer cover (2) comprises corresponding fastening surfaces (to the disposable absorbent insert as will be further described in embodiments hereinbelow) with the proviso that within the crotch region (4) said respective fastening surface is present only on one of the two crotch halfs, preferably said corresponding fastening surfaces comprising one or more woven, nonwoven or loop material.

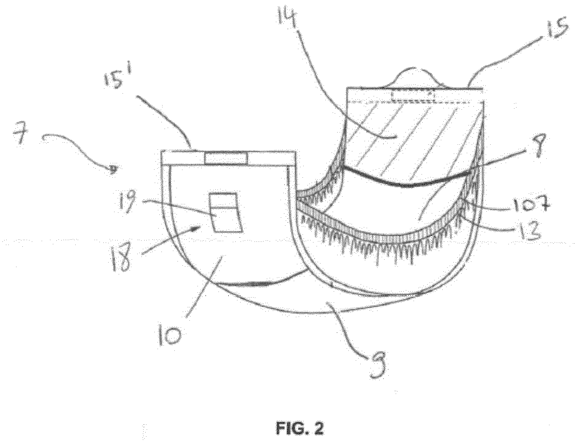

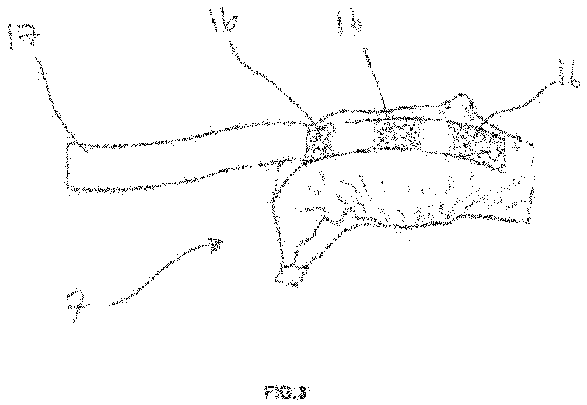

Referring to Figs.2-3, disposable absorbent inserts (7) according to embodiments herein and for use in an absorbent assembly (1) described herein typically comprise: a liquid permeable topsheet (8), a liquid impermeable backsheet (9), and an absorbent core (10) interposed between said topsheet (8) and said backsheet (9); a pair of longitudinal cuffs (13) extending along a length of the disposable absorbent insert (7); and a transversal barrier (14), typically extending in a direction substantially parallel to the transverse axis x, extending between oppositely extending lateral edges of said disposable absorbent insert (7), arranged on at least one end (15) thereof, wherein said transversal barrier (14) comprises indicia such that said disposable absorbent insert (7) may be joined to the elastic outer cover (2) of said absorbent assembly (1) in the correct front-to-back orientation. Advantageously this allows the user to correctly apply the insert into the outer cover (2) in the correct orientation. Indeed absorbent inserts are typically designed with graded absorbent properties along the length of the core and thus correctly orienting the insert is important to ensure efficacious exudate collection.

The absorbent core (10) may comprise absorbent material selected from the group consisting of cellulose fluff, cellulose fibers, and superabsorbent polymer particles.

In an embodiment, said insert (7) comprises a transversal barrier (14) having indicia in the form of a color such that the insert (7) may be joined to the elastic outer cover (2) of said absorbent assembly (1) on the back portion thereof.

Preferably, said insert (7) comprises two transversal barriers (14) oppositely disposed on each ends (15, 15') with the proviso that the barrier (14) proximal to the end (15), that is to be positioned on a back portion of the elastic cover (2) of said absorbent assembly (1), comprises indicia that is different to that of the oppositely disposed barrier, wherein said indicia is selected from the group consisting of a color and length, preferably wherein said indicia is the length of said barriers and wherein said barrier (14) proximal to said end (15) has a longer length, preferably being at least 1.5 times the length, of the other oppositely disposed barrier. Advantageously this allows to provide an orientation indication and also a functional barrier to liquid with the same components, indeed the barriers arranged as described herein act synergistically to provide liquid leakage prevention in the waist region (particularly important when using a foreign insert in a reusable outer cover that is not integral with said insert) and at the same time an indication as to correct orientation of the insert for correct assembly and efficacious exudate collection.

In an embodiment, a garment-facing surface of the absorbent insert (7) comprises a plurality fastening surfaces arranged to releasably engage with the one or more fastening surfaces of said outer cover (2), wherein said plurality of fastening surfaces are in the form of strips (16) each being separated along the length of said absorbent insert (7), more preferably being in the form of at least three strips (16) arranged proximal to the ends (15. 15') of said insert (7) and at a position therebetween. Advantageously this allows for secure mounting of the insert on the outer cover whilst limiting cost compared to for example having a continuous fastening surface.

Preferably, the plurality fastening surfaces are covered with a single release strip (17) that must be peeled off in order to join the disposable absorbent insert (7) to the elastic outer cover (2). Advantageously this prevents the fastening surface from becoming soiled and/or reduce its adhering efficacy when storing or prior to joining to the outer cover and allows for simple single-handed removal thereof prior to attachment.

Preferably, the plurality fastening surfaces comprise an adhesive or mechanical fasteners such as hooks, preferably adhesive.

In a preferred embodiment, a garment-facing surface of the absorbent insert (7) comprises a single fastening surface arranged to releasably engage with the one or more fastening surfaces of said outer cover (2), wherein said single fastening surface is in the form of a nonwoven having basis weight of from 10 to 35 g/m² that is laminated onto a garment-facing surface of the backsheet (9) of said absorbent insert (7) and is arranged to engage with a plurality of mechanical fasteners comprising hooks arranged on a skin-facing surface of the outer cover (2). Advantageously, when this is combined with one or more hooks present on a skin-facing surface of the outer cover allows for good attachment of the insert whiles at the same time providing a softer touch and feel to the garment-facing side of the backsheet.

Preferably, the garment-facing surface of the disposable absorbent insert (7), preferably the garment-facing surface of the backsheet (9) thereof, comprises two oppositely disposed pockets (18) proximal each end (15, 15') and separated from each other in a length direction of said insert (7), and arranged to receive a thumb of a subject in one of said pockets (18) and the rest of the fingers of the same hand of the subject in the other of said pockets (18) such that the insert may be single-handedly removed from the outer cover (2). Advantageously this aids the user to correctly single-handedly grip the insert and works synergistically with the designed release opening of the outer cover to allow a simple and effective single-handed removal of the insert once soiled.

In an embodiment, each pocket (18) comprises a substrate joined to a garment facing surface of said insert (7), preferably the garment-facing surface of the backsheet (9) thereof, having an open end distal from respective end (15, 15') and proximal to a transverse centreline of said insert (7) that divides said insert (7) into two halves, preferably wherein said open end is the only open end of each said pockets (18). Advantageously this allows for constructing pockets having a relatively large surface area to comfortably accommodate respective portions of an user's hand.

In an embodiment, the substrate comprises a nonwoven substrate having a basis weight of greater than 10 g/m² and wherein said substrate is joined to a garment facing surface of said insert (7), preferably the garment-facing surface of the backsheet (9) thereof, by mechanical or adhesive bonding substantially along a perimeter thereof except for the open end distal from respective end (15, 15') and proximal to a transverse centreline of said insert (7). This arrangement has the advantage of providing sufficient mechanical resistance to prevent tearing when the subject inserts his/her hand within respective pockets.

In an embodiment, the insert (7) further comprises a disposal tape (19) that may be arranged on a garment-facing surface of said insert (7), preferably of the pocket (18), such that once the insert is single-handedly removed it may be rolled up and joined to retain any exudates in an enclosed manner therein. Preferably the disposal tape (19) is arranged proximal to an end (15') of the insert (7), even more preferably is arranged only proximal to said end (15'). Advantageously, this allows the insert, once removed, to be rolled up starting from one end (15) towards the other end (15') and then simply joining the garment-facing surface of the insert (7) to said disposal tape (19).

### THE METHOD OF SINGLE-HANDED MANIPULATION

Referring to Fig.1C, a method of removing a disposable absorbent insert (7) from an elastic outer cover (2) may comprise the steps of: providing an elastic outer cover (2) comprising a front and back waist region (3) having uppermost and lowermost edges, and a crotch region (4) interposed between the front and back waist region, wherein the uppermost edges of the waist region (3) form a waist opening (5) and the lowermost edges of the waist region (3) together with lateral extremities of the crotch region (4) form two oppositely disposed leg openings (6), and wherein said crotch region (4) comprises one or more re-fastenable members (11) such that said crotch region (4) is unitary when said re-fastenable members (11) are fastened and that said crotch region (4) is separated into two crotch halfs when said re-fastenable members (11) are unfastened to form a crotch-release opening (12); providing a disposable absorbent insert (7) comprising a liquid permeable topsheet (8), a liquid impermeable backsheet (9), and an absorbent core (10) interposed between said topsheet (8) and said backsheet (9), wherein a skin-facing surface of said outer cover (2) is releasably joined to garment-facing surface of the absorbent insert (7); opening said re-fastenable members (11); pulling at least one of the two crotch halfs away from a garment-facing surface of the disposable absorbent insert (7) to expose at least a portion thereof; single-handedly grab the garment-facing surface of the disposable absorbent insert (7) with a thumb at a position proximal to one end (15') and distal to another end (15) of said insert (7), and with the remaining fingers at an opposite position distal to one end (15') and proximal to the other end (15) of said insert (7); squeeze the thumb and remaining fingers together; and substantially concurrently extracting the absorbent insert (7) from the crotch-release opening (12). Advantageously this method allows for single-handed removal of the insert whilst containing the exudate material in a cup-shape throughout the entire removal procedure and thus limit soiling of clothes and/or other surfaces.

Preferably, the thumb and the remaining fingers of a subject are inserted into oppositely disposed pockets (18), on the garment-facing surface of the absorbent insert (7), prior to the step of squeezing the thumb and remaining fingers together. Advantageously this arrangement allows for better location of the fingers of the user and provides added support and grip to allow the removal operation to be carried out successfully.

In an embodiment, as shown in Fig.1A, the method of inserting the insert (7) onto said outer cover (2) is different from the method of removal thereof (e.g. as described above). Preferably wherein the method of inserting the insert comprises the steps of inserting the insert (7) through the waist opening (5), typically whilst the re-fastenable members (11) are in a fastened state, and joining the garment-facing side of said insert (7) to the skin-facing side of said outer cover (2).

It is supposed that the present invention is not restricted to any form of realization described previously and that some modifications can be added to the presented example of fabrication without reappraisal of the appended claims.

## Claims

1. An absorbent article for personal hygiene such as a diaper (100), or training pant, or incontinence insert, the absorbent article comprising:
a liquid permeable topsheet (8);
a liquid impermeable backsheet (9);
an absorbent core (10, 101) interposed between said topsheet (8) and said backsheet (9), together forming an absorbent article chassis (102) having a front edge (103), a back edge (104) and oppositely disposed side edges (105, 105') connecting said front and back edges (103, 104) and running along a longitudinal axis (y);
an elastic-material-comprising component selected from the group consisting of:
one or more cuffs extending substantially parallel to said side edges (105, 105') comprising one or more elastic elements (107);
one or more transversal barriers (108) extending substantially perpendicular to the longitudinal axis (y) and along said front and/or back edges (103, 104), said transversal barriers (108) comprising one or more elastic elements (107);
one or more, preferably at least two, side panels (109) comprising one or more elastic elements (107), said side panels being joined to said chassis (102) about a proximal end (110) thereof and having a distal end (111) extending away from said chassis (102) along a transverse axis (x) being substantially perpendicular to said longitudinal axis (y);
one or more elastic waistbands (112) positioned adjacent said front and/or back edges (103, 104) comprising one or more elastic elements (107);
and combinations thereof; and
a detection device (113) that can be removably attached to the absorbent article,
**characterized in that** the elastic elements (107) comprise a conductive material and/or coating and are in electrical communication with said detection device (113) that is arranged to provide a signal upon a change in resistance or capacitance in response to a change in at least one of extension, tension, and/or rupture, of said elastic element(s) (107).

2. An absorbent article according to Claim 1, wherein the detection device is further arranged to provide a signal upon a change in resistance or capacitance in response to an exudate coming in contact with said elastic element(s) (107) typically following a voiding event.

3. An absorbent article according to any of the preceding Claims, wherein the conductive material and/or coating comprises a material selected from the group consisting of graphene, graphite, poly(pyrrole)s (PPY), polyanilines (PANI), poly(thiophene)s (PT), poly(p-phenylene sulfide) (PPS), poly(p-phenylene) (PPP), Poly(acetylene)s (PAC), Poly(p-phenylene vinylene) (PPV), poly(3,4-ethylenedioxythiophene) (PEDOT), and mixtures thereof, more preferably poly(3,4-ethylenedioxythiophene) polystyrene sulfonate (PEDOT:PSS).

4. An absorbent article according to Claim 3, wherein the material is comprised at a level of from 10 to 45%, preferably from 15 to 35%, by weight of the total composition of the conductive material and/or coating.

5. An absorbent article according to any of the preceding Claims further comprising a conductive ink pattern applied, preferably printed, onto at least a portion of the chassis (102), preferably a skin-facing surface of the backsheet (9), and arranged such to form a conductive bridge between said elastic elements (107) and the detection device (113).

6. An absorbent article according to Claim 5 wherein the conductive ink pattern is in the form of an adhesive, preferably a stretchable adhesive.

7. An absorbent article according to Claims 5 to 6 wherein the conductive ink comprises a a material selected from the group consisting of graphene, graphite, poly(pyrrole)s (PPY), polyanilines (PANI), poly(thiophene)s (PT), poly(p-phenylene sulfide) (PPS), poly(p-phenylene) (PPP), Poly(acetylene)s (PAC), Poly(p-phenylene vinylene) (PPV), poly(3,4-ethylenedioxythiophene) (PEDOT), and mixtures thereof, more preferably poly(3,4-ethylenedioxythiophene) polystyrene sulfonate (PEDOT:PSS).

8. An absorbent article according to any of the preceding Claims wherein the one or more elastic elements (107) are strand coated with said conductive material and/or coating.

9. An absorbent article according to any of the preceding Claims wherein the detection device (113) comprises an integrated memory to store at least a portion of the data collected by a processor contained within the detection device (113) during a monitoring period, and a transmitter to transmit said data at regular predetermined periods of time to a data receiver for further processing of said data.

10. An absorbent article according to any of the preceding Claims wherein the absorbent article comprises a disposable insert (107) and a washable and re-usable outer shell (2), wherein the disposable insert (7) comprises the liquid permeable topsheet, the liquid impermeable backsheet and the absorbent core, and wherein the washable and re-usable outer shell (2) comprises a second liquid permeable woven or nonwoven backsheet (4) to which the elastic-material-comprising components are joined.

11. An absorbent article according to Claims 1 to 9, wherein the absorbent article comprises a disposable insert (107) and a washable and re-usable outer shell (2), wherein the disposable insert (7) comprises the liquid permeable topsheet, a liquid permeable backsheet and the absorbent core positioned between said permeable topsheet and permeable backsheet, and wherein the washable and re-usable outer shell (2) comprises a second backsheet being the liquid impermeable backsheet (4) to which the elastic-material-comprising components are joined.

12. An absorbent article according to Claims 10 to 11, wherein the disposable insert (107) further comprises a pair of longitudinally extending inner cuffs (13) and wherein the reusable outer shell comprises a second pair of longitudinally extending outer cuffs (106) that are positioned outboard the inner cuffs (13) in assembled state, preferably wherein the outer cuffs (106) comprise the elastic elements (107) comprising a conductive material and/or coating, and preferably wherein the disposable insert is free of conductive material and/or coating.

13. An absorbent article according to Claims 10 to 12 wherein the second backsheet (4), preferably a garment facing surface of said second backsheet, comprises the conductive ink pattern of Claim 4, preferably wherein said pattern extends from a front edge to a back edge of said backsheet.

14. An absorbent article according to any of the preceding Claims wherein the elastic element (107) comprises a conductive yarn or fiber.

15. An absorbent article according to Claims 1 to 13 wherein the elastic element (107) comprises a conductive filler, preferably wherein the conductive filler has a melting temperature that is higher than the melting temperature of one or more polymers forming said elastic element.

16. An absorbent article according to Claim 15 wherein the conductive filler is selected from the group consisting of graphene, graphite, and mixtures thereof, preferably wherein said filler is comprised at a level of from 5% to 30%, preferably from 10% to 25%, by weight of the elastic element.

17. A kit of parts comprising a washable and re-usable outer shell (2) according to Claims 10 to 16, a plurality of disposable inserts (7) according to Claims 10 to 16 and at least one detection device (113).
